# FASCICULE DE BREVET EUROPEEN

(11) **EP 0 593 321 B1**
(45) Date de publication et mention de la délivrance du brevet: **27.10.1999**
(21) Numéro de dépôt: 93402234.4
(22) Date de dépôt: 14.09.1993
(51) Int. Cl.: A61B 17/70

(54) **Système d'ostéosynthèse rachidienne**
Vorrichtung zur Osteosynthese von Rückenwirbeln
Device for spinal osteosynthesis

(30) Priorité: 15.09.1992 FR 9210956
(43) Date de publication de la demande: 20.04.1994
(73) Titulaire: Cavagna, Rémi, 56270 Ploemeur (FR); Clerc, Robert, 22100 Dinan (FR)
(72) Inventeur: Cavagna, Rémi, 56270 Ploemeur (FR); Clerc, Robert, 22100 Dinan (FR)
(74) Mandataire: Hirsch, Marc-Roger

(56) Documents cités:
- EP-A- 0 348 581
- EP-A- 0 383 992
- EP-A- 0 443 894
- DE-A- 3 924 050
- FR-A- 2 615 095
- GB-A- 2 178 323
- US-A- 5 127 912

## Description

La présente invention a pour objet un nouveau système d'ostéosynthèse rachidienne qui est polyvalent, simplifié, et rend possible une action déterminante du matériel ancillaire. La présente invention se rapporte aussi à la technique de mise en place dudit système d'ostéosynthèse.

L'ostéosynthèse du rachis dorsal, lombaire et sacré existe depuis longtemps. On fait appel à cette technique dans plusieurs indications thérapeutiques: traumatologie; rachis dégénératif, malformatif, tumoral; scoliose primitive ou secondaire; instabilité rachidienne; et autres.

Les cas pathologiques qui nécessitent donc l'ostéosynthèse sont nombreux. Dans un premier temps, on considère les déviations qui sont des anomalies particulièrement fréquentes: accentuation de la cyphose dorsale, de la lordose lombaire et surtout de la scoliose, ou déviation latérale du rachis. Les cas graves de scolioses osseuses ou structurelles, qui s'accompagnent d'une rotation des vertèbres autour de leur axe vertical sont d'origines diverses. Ces scolioses peuvent être congénitale, endocrinienne, neurologique (poliomyélite) ou musculaire (myopathie). Parfois, il n'existe pas de cause évidente, on parle de scoliose essentielle (de l'adolescent). Dans un second temps, on considère les déviations dues à des traumatismes du rachis, très souvent à la suite d'un accident de la circulation. Les fractures du rachis sans lésion médullaire (ou de la moelle épinière) requièrent, dans les cas graves, une immobilisation du rachis par ostéosynthèse. Dans le cas de fractures du rachis avec lésion médullaire, l'immobilisation par ostéosynthèse se révèle utile, particulièrement dans le cas où il y a seulement contusion ou compression médullaire, c'est-à-dire dans les cas où il existe une possibilité de régression. Enfin, on considère que le recours à l'ostéosynthèse est efficace aussi dans le cas de tumeurs malignes. L'ostéosynthèse est aussi utilisée dans le cas d'ostéoporose ou d'épiphysite vertébrale, qui se traduisent sur le plan du rachis par l'apparition de cyphose accompagnant des déformations vertébrales (vertèbres cunéiformes, trapézoïdales). On a aussi recours à l'ostéosynthèse dans les cas graves d'arthrose. Il est donc évident qu'il existe un besoin en matériel d'ostéosynthèse rachidienne, car les cas pathologiques qui nécessitent un tel matériel sont nombreux.

De nombreux systèmes d'ostéosynthèse ont été proposés jusqu'à présent. Le matériel de Harrington, un des plus anciens, se compose de crochets sus- et sous-lamaires et de tiges. L'appui vertébral se fait au niveau des deux extrémités et la réduction ne peut se faire qu'en distraction. Ce matériel ancien présente des inconvénients notables: faibles possibilités de manoeuvres, faible tenue aux extrémités et un encombrement important. Le matériel de Cotrel a marqué une évolution notable et il permet une fixation plus courte, plus stable, et des manoeuvres de réduction plus complexes. Ce matériel comprend des crochets sous-pédiculaires d'un type nouveau, des vis pédiculaires, éventuellement dites "vis tulipe", des tiges ayant une surface diamantée, et un dispositif de traction/renforcement de traction transversale. Néanmoins, ce matériel, bien qu'étant une évolution sensible par rapport au précédent, présente toujours des inconvénients majeurs. Ainsi, outre la complexité du matériel de Cotrel, les manoeuvres de réduction sur ce matériel s'effectuent par l'intermédiaire des tiges préalablement moulées sur les points d'ancrage, crochet ou vis. Ces manoeuvres peuvent conduire à la rupture des points d'appui, due à une sollicitation de l'os trop importante, ou peuvent avoir comme résultat des réductions imparfaites, par excès ou par défaut. D'autres systèmes ont aussi été développés, et on peut citer le système de plaques vissées, dont le précurseur est le système de Roy-Camille. Celui-ci repose sur le même principe de réduction sur les points d'ancrage à partir de plaques déjà formées et conduit donc aux inconvénients précités pour le système Cotrel. Ces systèmes, plus ou moins anciens, montrent parfois des points de faiblesse et des ruptures de matériels, probablement en rapport avec les excès de contraintes mécaniques. Le système Wiltse comprend une vis pédiculaire, un étrier chevauchant cette vis et recevant dans un premier temps une matrice en aluminium malléable qui est ensuite reproduite sous forme d'une tige s'adaptant au profit dessiné par les positions des diverses vis sur le rachis, un système de fixation par écrou qui solidarise vis et tige avant l'action mécanique de réduction exercée par le chirurgien. Ce système présente les inconvénients mentionnés précédemment pour le système de Cotrel, dont il reprend par ailleurs le principe.

US-A-5 127 912 décrit un système d'implant sacré, permettant d'obtenir une fixation particulièrement solide au niveau du sacrum, et de fixer la partie supérieure de l'implant sur la vertèbre L1 sans interférence avec la vertèbre immédiatement supérieure. Ce système est destiné à être mis en place comme les systèmes décrits plus hauts. EP-A-0 443 894 décrit un dispositif d'ostéosynthèse pour la correction des déviations rachidienne, dont la mise en place s'effectue aussi avant d'exercer les actions mécaniques de réduction.

En fait, ces systèmes selon l'art antérieur présentent de nombreux problèmes. Certains matériels doivent être posés selon le plan sagittal; ceci pose évidemment le problème de l'encombrement stérique et des difficultés que rencontre le chirurgien pour accéder à la zone opératoire. Mais surtout, les matériels classiques présentent des inconvénients dus à la méthode jusqu'à présent employée pour poser ceux-ci. En effet, la séquence opératoire utilisée jusqu'à maintenant est la suivante: fixation des vis, éventuellement fixation de manière solidaire des plaques transversales, fixation de la tige (éventuellement préformée grâce à une matrice en alliage malléable permettant de reproduire le positionnement des vis selon la pathologie) solidairement sur lesdites vis, puis action mécanique à l'aide d'un matériel ancillaire. Cette séquence présente trois inconvénients majeurs. Tout d'abord, la tige qui est initialement posée se déforme et la forme finale qu'elle doit avoir n'est pas toujours atteignable, les vertèbres restant en hypo- ou hyper-réduction. Les contraintes mécaniques sont alors élevées et il est parfois nécessaire de changer la tige en cours d'opération et de la remplacer par une tige ayant une forme intermédiaire. Ceci peut être critique dans certains cas opératoires délicats comme, par exemple, ceux faisant intervenir un appui iliaque. Le second problème que présentent ces systèmes d'ostéosynthèse selon l'art antérieur est que l'action mécanique exercée par le chirurgien est la somme de l'action mécanique sur le système osseux et de l'action sur le système d'ostéosynthèse lui-même. Ainsi, le chirurgien n'est pas toujours à même d'apprécier les contraintes qu'il exerce sur les vertèbres; chez les sujets âgées, la fragilité des vertèbres peut alors conduire à un arrachement des points d'ancrage, ce qui résulte finalement en un ancrage sur la vertèbre suivante, augmentant la longueur du système d'ostéosynthèse et, par là-même, les gênes qu'il occasionne chez le patient. Enfin, la réduction du rachis est basée sur la combinaison d'action mécanique selon deux degrés de liberté. Le praticien doit, dans certains cas, exercer des actions de rotation-dérotation, tandis que, dans d'autres cas, il doit exercer des actions de compression-distraction, voire aussi des actions de tire-fond. Aucun système actuel ne permet d'effectuer ces actions mécaniques indépendantes de façon satisfaisante.

Il existe donc un besoin d'un système d'ostéosynthèse rachidienne qui permet le travail dans le plan frontal, des actions mécaniques sur le matériel et les os séparées, une fiabilité conduisant immédiatement et sûrement à la forme de tige requise, et qui permette des actions de rotation-dérotation, de compression-distraction, et de tire-fond aisées. Cet objet, ainsi que d'autres, est atteint par la présente invention.

Ainsi, la présente invention fournit un système d'ostéosynthèse rachidienne comprenant:
- une vis pédiculaire munie de deux filetages, le premier filetage, le filetage pédiculaire, étant destiné à être introduit dans le pédicule vertébral et le second filetage, le filetage de platine, étant destiné à recevoir la platine;
- une tige;
- au moins une platine constituée d'au moins une tête perforée d'un trou dont le diamètre est sensiblement égal à celui du filetage de platine de la vis pédiculaire et au moins une gorge dont l'axe est sensiblement perpendiculaire à l'axe de la tête;
- ladite gorge comprenant un alésage fendu dont l'espace de l'ouverture correspond au moins au diamètre de la tige;
- un moyen de fixation de la platine sur la vis destiné à solidariser ladite platine au niveau de la tête sur ladite vis pédiculaire;
- lesdits moyen de fixation et filetage de platine étant tels que le moyen de fixation engagé à blocage laisse libre une partie du filetage de platine permettant ainsi le fixation sur 'l'extrémité libre d'un matériel ancillaire utilisé pour une action mécanique de réduction;
- un moyen de fixation de la tige dans la gorge destiné à solidariser ladite tige dans ladite gorge de ladite platine.

Le nouveau système d'ostéosynthèse rachidienne selon la présente invention permet au chirurgien d'intervenir sur le rachis de façon plus simple, plus efficace et plus sûre. En fait, le présent système d'ostéosynthèse rachidienne permet la séquence opératoire suivante:
a) fixation des vis pédiculaires;
b) fixation de la platine, comprenant une tête et une gorge, solidairement sur la vis pédiculaire par introduction sur la vis pédiculaire de la tête perforée d'un trou d'un diamètre sensiblement égal à celui du filetage de platine de la vis pédiculaire, puis blocage par un écrou sur le filetage de platine, ledit écrou une fois en position bloquée laissant une partie du filetage de platine libre;
c) action mécanique sur les vis pédiculaires à l'aide d'un matériel ancillaire constitué d'un tube de réduction et de pinces pour distraction, contraction et (dé)rotation dont le mors à l'extrémité distale a la forme générale d'un croissant de lune s'adaptant au tube précité; subséquemment, blocage en cette position par des "clamps";
d) à l'issue de l'action mécanique, prise de l'empreinte des vis et platines et fabrication d'une tige correspondante;
e) mise en place de ladite tige préformée sur l'ensemble des vis, ladite tige étant insérée dans l'alésage fendu de ladite gorge et un moyen de fixation de la tige dans la gorge est alors mis en oeuvre pour obtenir le blocage de l'ensemble qui forme le système d'ostéosynthèse rachidienne en position définitive;
f) retrait du matériel ancillaire et terminaison de l'intervention chirurgicale.

Cette séquence opératoire, rendue possible grâce au nouveau système d'ostéosynthèse rachidienne selon la présente invention permet donc une action mécanique uniquement sur les os. Le chirurgien, ne prenant en compte que les contraintes du système osseux, peut donc éviter une action mécanique trop importante qui pouvait conduire, dans certains cas pathologiques, avec des patients âgés par exemple et avec le matériel selon l'art antérieur, à des bris de matériel, le plus souvent d'os ainsi qu'à des arrachements de points d'ancrage, ce qui obligeait à poser le matériel sur la vertèbre suivante. De plus, la tige préformée est la réplique de la tige en position finale, alors que selon l'art antérieur, on exerçait une action sur le matériel complet, c'est-à-dire avec la tige, et à l'issue de l'action mécanique de réduction, la tige pouvait se révéler inadaptée car trop courte ou trop longue, ou subissant des contraintes mécaniques trop importantes, et on pouvait alors soit laisser le patient avec une hypo- ou une hyper-réduction, soit on devait recommencer en partant d'une position intermédiaire, ce qui nécessitait un temps d'intervention et d'anesthésie plus long. Enfin, le matériel selon l'invention est parfaitement adapté à une intervention dans le plan frontal; ce qui permet une plus grande facilité de mouvement; cependant, il est possible de poser le nouveau système d'ostéosynthèse rachidienne selon la présente invention dans le plan sagittal.

En fait, les inconvénients selon l'art antérieur dérivent du fait que la séquence opératoire utilisée était, avec la nomenclature ci-dessus, et pour un système du type Cotrel, Wiltse, ou autres: (a), (b), (d), (e), (c), (f). La séquence selon l'art antérieur est en fait:
a) fixation des vis pédiculaires;
b) fixation de la platine, comprenant une tête et une gorge, solidairement sur la vis pédiculaire par introduction sur la vis pédiculaire de la tête perforée d'un trou d'un diamètre sensiblement égal à celui du filetage de platine de la vis pédiculaire, puis blocage par un écrou sur le filetage de platine, ledit écrou une fois en position bloquée laissant une partie du filetage de platine libre;
d) prise de l'empreinte des vis et platines et fabrication d'une tige préformée correspondante ayant la forme de la pathologie;
e) mise en place de ladite tige préformée sur l'ensemble des vis, ladite tige étant insérée dans l'alésage fendu de ladite gorge et un moyen de fixation de la tige dans la gorge est alors mis en oeuvre pour obtenir le blocage de l'ensemble qui forme le système d'ostéosynthèse rachidienne, ce dernier constituant alors un système mécanique hyperstatique;
c) action mécanique sur ce système mécanique, par l'intermédiaire des vis pédiculaires, à l'aide d'un matériel ancillaire constitué d'un tube de réduction et de pinces pour distraction, contraction et dérotation dont le mors à l'extrémité distale a la forme générale d'un croissant de lune s'adaptant au tube précité;
f) retrait du matériel ancillaire et terminaison de l'intervention chirurgicale.
Grâce au présent système d'ostéosynthèse rachidienne, la séquence selon l'invention est permise.

Il est possible de fournir de multiples variantes du présent système d'ostéosynthèse rachidienne, toutes aisément accessibles à l'homme de l'art et qui permettent une adaptation à chaque cas pathologique.

Dans la présente vis pédiculaire, le filetage de platine est à pas faible tandis que le filetage pédiculaire présente un pas large.

La vis pédiculaire est munie d'un moyen de serrage approprié qui permet de visser cette vis pédiculaire dans l'os pédiculaire. De préférence, ce moyen de serrage est situé entre les deux filetages.

Selon un mode de réalisation, ce moyen de serrage est un écrou hexagonal situé entre le filetage de platine et le filetage pédiculaire. Avantageusement, au moins un des pans de l'écrou hexagonal correspond avec un méplat présent sur la tête de la platine.

L'alésage fendu peut avoir toute forme possible qui permette à la tige d'être introduite dans la gorge et fixée solidairement dans celle-ci.
Selon un mode de réalisation, l'alésage fendu a la forme d'un U tandis que selon une autre variante, ledit alésage a la forme d'un Ω (oméga).

Tout moyen de fixation de la platine sur la vis pédiculaire est approprié; néanmoins on préfére un écrou.

Tout système de fixation de la tige dans la gorge de la platine est approprié, selon les formes respectives de la gorge et de la tige. Il est possible, par exemple, d'utiliser une vis dont le diamètre est croissant et qui est introduite dans l'espace laissé libre entre la tige et l'alésage fendu. Lors du vissage de la vis, cette dernière, du fait de son diamètre croissant, exerce alors une contrainte mécanique qui permet de bloquer tige et gorge de la platine; ce type de fixation est par ailleurs déjà utilisé, notamment au cours de l'opération de ligamentoplastie de remplacement du ligament croisé antérieur. Tout autre système est adapté, notamment le système de bagues enfilées sur la tige, ou encore de clavette.

Selon un mode de réalisation, avantageusement lorsque l'alésage fendu a la forme d'un Ω (oméga), le moyen de fixation de la tige dans la gorge est une clavette excentrique dont le diamètre interne est substantiellement identique à celui de la tige et dont le diamètre externe est tel qu'une action mécanique de contrainte est exercée sur la platine lors de la rotation ou translation de ladite clavette.

Selon un autre mode de réalisation, lorsque l'alésage a la forme d'un U, le moyen de fixation de la tige dans la gorge est une vis s'engageant dans un filetage ménagé dans l'alésage de la gorge, sensiblement perpendiculairement à cette dernière et sensiblement parallèlement à la vis pédiculaire. De préférence, la vis de fixation est une vis sans tête.

La vis pédiculaire peut par ailleurs comporter d'autres éléments mécaniques ayant des fonctions définies. Ainsi, la vis comporte avantageusement un moyen de limiter sa course afin que seule la longueur nécessaire de la vis soit placée dans le pédicule de la vertèbre, et que la zone médullaire ne subisse ni lésion, ni compression.
Avantageusement, la vis comporte une couronne externe entre les filetages afin de placer la vis pédiculaire en butée sur le rachis.

Selon un mode de réalisation de la présente invention, la vis pédiculaire est de section décroissante dans sa partie pédiculaire. Avantageusement, le filetage pédiculaire possède un profil à fond plat.

Tout écrou est approprié pour fixer la platine sur la vis pédiculaire; cependant on donne la préférence à un écrou portant des rainures, destinées à recevoir et à guider le moyen de serrage.

On cherchera aussi à éviter la rotation de la platine sur la vis pendant l'étape de serrage de l'écrou qui bloque la platine sur la vis. Tout système mécanique à cette fin peut être utilisé, par exemple une canelure taillée dans le filetage de platine, et qui correspond à une saillie située sur la périphérie du trou de la tête de la platine.
Selon un mode de réalisation, la tête de la platine comporte un méplat et la vis pédiculaire comporte sur la couronne externe entre les deux filetages un méplat, lesdits méplats étant correspondants et s'engageant afin d'empêcher toute rotation de la platine sur la vis.
Selon un autre mode de réalisation, la tête de la platine comporte un méplat et la vis pédiculaire comporte un écrou hexagonal situé entre le filetage de platine et le filetage pédiculaire, ledit méplat et au moins un pan de l'écrou héxagonal étant correspondants et s'engageant afin d'empêcher toute rotation de la platine sur la vis.
Selon un autre mode de réalisation, la couronne externe située entre les deux filetages de la vis pédiculaire est une couronne dentée s'engageant dans une couronne dentée correspondante sur la platine, afin d'empêcher toute rotation de la platine sur la vis.

Le système d'ostéosynthèse selon la présente invention peut être posé sur le rachis sur un côté seulement, ou sur les deux côtés. Dans ce dernier cas, le système selon la présente invention intègre un élément mécanique approprié afin de relier, si on le souhaite, les deux vis posées sur une même vertèbre.
De préférence, cet élément de liaison est une plaque transversale s'étendant entre deux vis pédiculaires sur une même vertèbre, fixée solidairement sur celles-ci avec les deux platines correspondantes et les deux écrous correspondants, avant action mécanique de réduction. Les trous aux extrémités de la plaque transversale sont avantageusement ovalaires, de sorte à n'employer qu'une plaque transversale pouvant s'adapter à diverses conformations osseuses de vertèbres différentes. La plaque peut être posée après réduction pour réaliser un montage dit "montage en cadre".

La platine possède au moins une gorge, mais il n'y a aucune contre-indication à ce que la platine soit munie de plus d'une gorge. Selon un mode de réalisation de la présente invention, la platine possède deux gorges. Cette platine à deux gorges peut être utilisée, par exemple, en lieu et place des dominos, habituellement utilisés pour joindre deux tiges distinctes.

Par ailleurs, la vis pédiculaire n'est pas limitée à porter une seule platine. Selon un mode de réalisation de la présente invention, le système d'ostéosynthèse rachidienne comprend deux platines, inférieure et supérieure. La présence de ces deux platines permet de générer deux axes de renforcement, par exemple, de substituer la plaque transversale par une tige similaire à celle utilisée longitudinalement au rachis.

Selon ce mode de réalisation, les platines sont solidarisées toujours avec un écrou et il est souhaitable d'éviter leur rotation l'une par rapport à l'autre, de la mâme façon que précédemment, par rapport à la vis pédiculaire. Selon un mode d'exécution de cette variante de l'invention, la platine inférieure possède au moins un méplat supérieur sur la tête, la platine supérieure possède un méplat inférieur, lesdits méplats supérieur de la platine inférieure et inférieur de la platine supérieure venant en engagement. Selon un autre mode d'exécution de cette variante de l'invention, la platine inférieure possède au moins une couronne dentée supérieure sur la tête, la platine supérieure possède une couronne dentée inférieure, lesdites couronnes dentées supérieure de la platine inférieure et inférieure de la platine supérieure venant en engagement.

Ce nouveau système d'ostéosynthèse rachidienne est donc parfaitement adapté à une utilisation selon la nouvelle séquence opératoire chirurgicale. Ainsi, la présente invention a aussi pour objet l'utilisation du système d'osthéosynthèse rachidienne pour la réduction de rachis avec action mécanique de réduction antérieure à la fixation des tiges sur les vis pédiculaires.

La présente invention est maintenant décrite plus en détail en référence aux dessins des figures suivantes:
- la figure 1a est une vue d'une vis pédiculaire selon la présente invention;
- la figure 1b est une vue en élévation de la même vis;
- la figure 2a est une vue de face de l'écrou pédiculaire;
- la figure 2b est une vue de dessus de l'écrou pédiculaire;
- la figure 3a est une vue de face d'une platine selon un premier mode de réalisation de la présente invention;
- la figure 3b est une vue de dessus de la platine représentée à la figure 3a;
- la figure 3c est une vue de la coupe identifiée par AA sur la figure 3a;
- la figure 4 est une vue de la vis sans tête de blocage de la tige dans l'alésage;
- la figure 5 est une vue en perspective de la platine selon un autre mode de réalisation de l'invention;
- la figure 6a est une vue d'une couronne sur la vis pédiculaire;
- la figure 6b représente la tête de platine s'engageant sur la couronne représentée à la figure précédente;
- la figure 7 est une vue en perspective d'un moyen de fixation selon un mode de réalisation de l'invention;
- la figure 8 est une vue de la plaque transversale selon l'invention;
- la figure 9 est une vue en perspective du système d'ostéosynthèse selon l'invention;
- la figure 10 représente un tube de réduction utilisé pour la mise en oeuvre de l'invention.

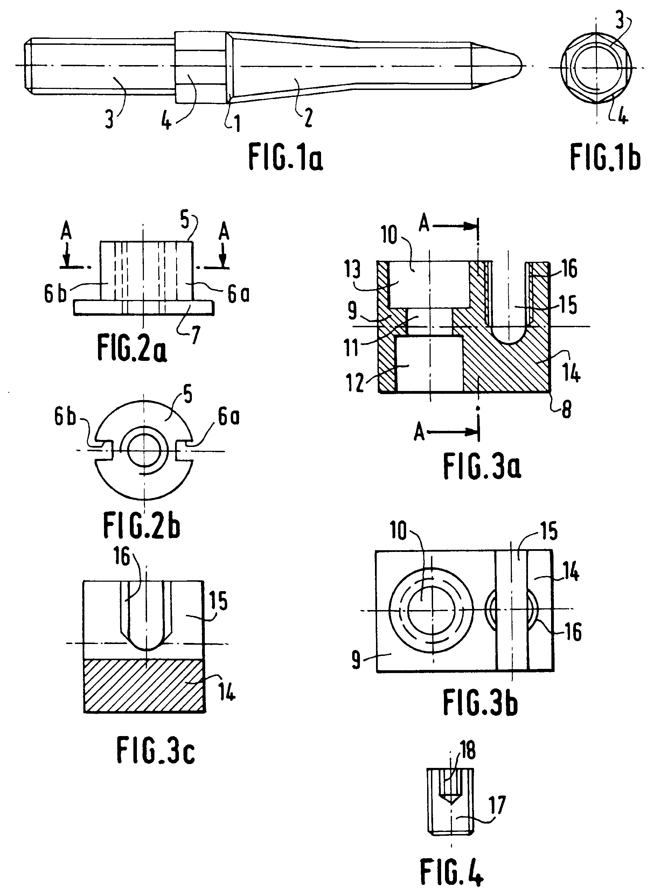

La figure la est une représentation d'une vis pédiculaire 1 selon l'invention. Cette vis 1 comporte deux filetages, le premier filetage 2 pédiculaire à pas large étant introduit dans la partie osseuse de la vertèbre au niveau du pédicule. Le second filetage 3 de platine à pas faible est situé dans la partie extra-osseuse de la vis pédiculaire. Ce dernier filetage est destiné à recevoir, d'une part, la platine et l'écrou de blocage, éventuellement la plaque transversale, et d'autre part, le matériel ancillaire constitué des tubes et pinces définis ci-avant. De préférence, la partie intra-osseuse, c'est-à-dire la partie portant le filetage pédiculaire, est de forme globalement conique dans sa partie supérieure et globalement cylindrique dans sa partie inférieure, comme illustrée précisément sur la figure 1. Cependant, une forme cylindrique sur toute la longueur ou toute autre forme est appropriée. La vis 1 peut comporter une couronne (non représentée), dont une fonction est d'assurer la mise en butée de la vis pédiculaire sur l'os. La vis comporte un moyen de serrage 4. Celui-ci peut être une entaille au sommet du filetage de platine, de façon à créer un espace permettant d'insérer un tournevis plat, cruciforme, hexagonal. De préférence, ce moyen de serrage 4 a la forme générale d'un écrou à 6 pans plats permettant de recevoir une clé de serrage. Tout autre moyen de serrage peut être utilisé, par exemple une cavité hexagonale qui reçoit un tournevis de montage dont l'extrêmité s'adapte à la cavité hexagonale sus-citée.

La figure 1b est une vue en élévation de la vis 1 selon la présente invention. On distingue nettement le profil à 6 pans plats du moyen de serrage 4 ainsi que le filetage 3 de platine.

La figure 2a est une vue de face de l'écrou pédiculaire ou écrou de blocage 5 de la platine. Le diamètre interne et la pas de vis de l'écrou 5 correspondent aux diamètre et pas du filetage de platine 3. Cet écrou 5 possède selon le mode de réalisation représenté deux rainures latérales 6a et 6b destinées à recevoir l'instrument de serrage. Il possède aussi une couronne 7 qui se trouve au niveau de la zone de contact.

La figure 2b est une vue selon la coupe AA donnée à la figure 2a du même écrou pédiculaire.

La figure 3a est une vue de face d'une platine selon un mode de réalisation de la présente invention. Cette platine 8 comporte une tête 9 percée d'un trou 10. L'extrêmité médiane 11 de ce trou est d'un diamètre similaire à celui du filetage 3 de platine, filetage sur lequel la platine vient s'engager. La partie inférieure 12 du trou, i.e. la partie la plus proche de l'os, possède un diamètre et une profondeur suffisants pour s'adapter et venir chapeauter le moyen 4 de serrage, représenté par un profil 6 à pans plats. La partie supérieure 13 du trou, i.e. la partie la plus éloignée de l'os, possède un diamètre et une profondeur suffisants pour s'adapter à l'écrou pédiculaire 5. La platine comprend en outre, disposée substantiellement perpendiculairement à la tête, une gorge 14. Cette gorge 14 comprend un alésage fendu 15, dont le diamètre correspond sensiblement au diamètre de la tige que l'alésage reçoit. Selon le mode de réalisation représenté sur la figure, l'alésage a la forme d'un U. Cet alésage possède en outre un filetage 16, disposé perpendiculairement à l'alésage et sensiblement parallèle à l'axe du trou 10, destiné à recevoir la vis sans tête de blocage 17.

La figure 3b est une vue de dessus de la platine 8, dans laquelle on retrouve les éléments suivants: tête 9, trou 10, gorge 14, alésage 15 et filetage 16.

La figure 3c est une vue de la coupe identifiée comme AA sur la figure 3a. Cette coupe est réalisée dans la gorge 14 au niveau de l'alésage 15 et réprésente plus en détail le filetage 16. Ce dernier s'étend sur toute la profondeur de l'alésage, bien que cela ne soit pas critique pour la réalisation de l'invention, qui est atteinte lorsque le filetage s'étend sur une longueur suffisante pour que l'extrêmité de la vis de blocage soit en contact serré avec la tige.

La figure 4 est une vue de la vis sans tête de blocage 17, qui s'engage dans le filetage 16 prévu dans l'alésage 15. Cette vis sans tête possède un évidement 18 ayant une forme hexagonale, type à 6 pans plats, permettant de recevoir un outil pour le serrage de la vis de blocage.

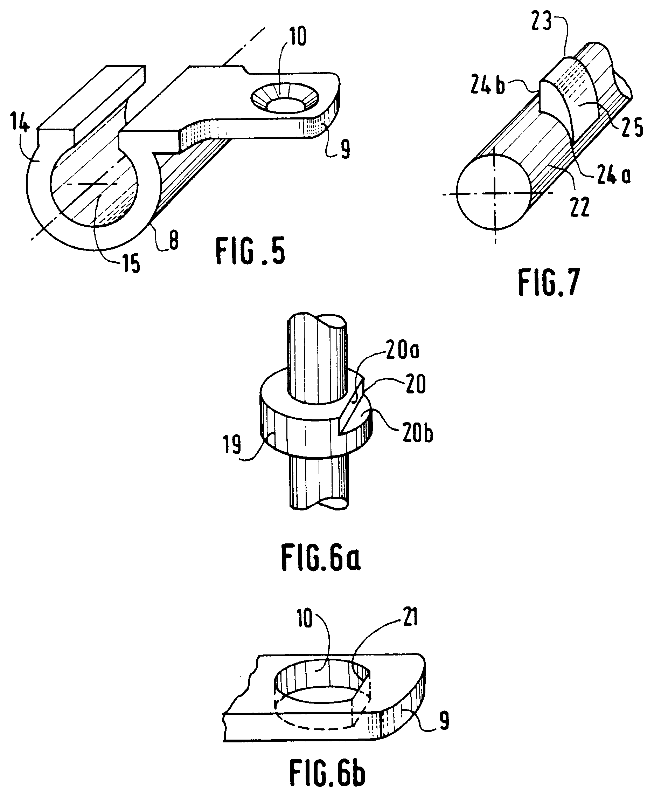

La figure 5 est une vue en perspective de la platine 8 selon un autre mode de réalisation de l'invention. Les éléments dont seulement des variantes d'éxécution sont représentées sont numérotés de façon identique. Cette platine 8 comprend une tête 9, comportant un trou 10 dont le diamètre correspond à celui du filetage de platine. La platine comprend en outre, disposée substantiellement perpendiculairement à la tête, une gorge 14. Cette gorge 14 comprend un alésage fendu 15, dont l'ouverture correspond au diamètre de la tige. Le mode réalisation représenté est celui dans lequel l'alésage fendu possède la forme d'un Ω (oméga), qui est "retourné" sur la figure.

La figure 6a est une vue agrandie d'une couronne 19, lorsque celle-ci est présente, de la vis 1. Cette couronne 19 possède un méplat 20, constitué d'une face 20a parallèle à l'axe de la vis et d'une face 20b transverse ou perpendiculaire à l'axe de la vis. Ce méplat s'engage en correspondance dans la tête de la platine afin d'empêcher toute rotation de la platine autour de la vis. En fait, la couronne est optionnelle lorsque le moyen de serrage 4 possède un profil à 6 pans plats, puisque dans ce dernier cas le moyen de serrage présente la face plane 20a.

La figure 6b est une vue agrandie de la tête de la platine qui s'adapte à la couronne décrite figure 6a. La tête 9 possède, dans la partie inférieure du trou 10, aussi un méplat 20 dont la partie plane correspond à la face 20a sur le méplat 21 de la vis 1, ou correspond à un des pans du moyen de serrage 4 lorsque ce dernier est celui représenté à la figure la.

La figure 7 est une vue en perspective d'un moyen de fixation selon un mode de réalisation de la présente invention. Ce moyen de fixation est posé sur la tige 22 et est appelé dans ce qui suit clavette. Cette clavette 23 a un diamètre intérieur sensiblement égal au diamètre de la tige et recouvre cette dernière sur substantiellement le quart de sa périphérie. Un recouvrement supérieur à la moitié conduisant à un léger effet de pincement de la clavette, par exemple dans le cas où la clavette est symétrique, peut être cependant recherché dans certains cas. La clavette 23 peut tourner autour de la tige 22, grâce à un matériel qui exerce une force de rotation en prenant appui sur les faces 24a et 24b (cachée sur la figure). La clavette est excentrique et possède une face 25 présentant un profil convexe et qui s'étend de la face 24a et de la face 24b (non représenté sur la figure). Cette clavette, dont les dimensions sont adaptées, provoque, lors de sa rotation dans l'alésage 15 de la platine, l'écartement de ce dernier et les efforts de contraintes bloquent tige, clavette et platine dans une position définitive. Cette rotation de la clavette dans l'alésage est rendue possible par la face 25 convexe de la clavette qui n'oppose qu'un frottement faible lors de la rotation. l'angle aigu formé par les faces 25 et 24b empêche toute rotation ultérieure de la clavette.

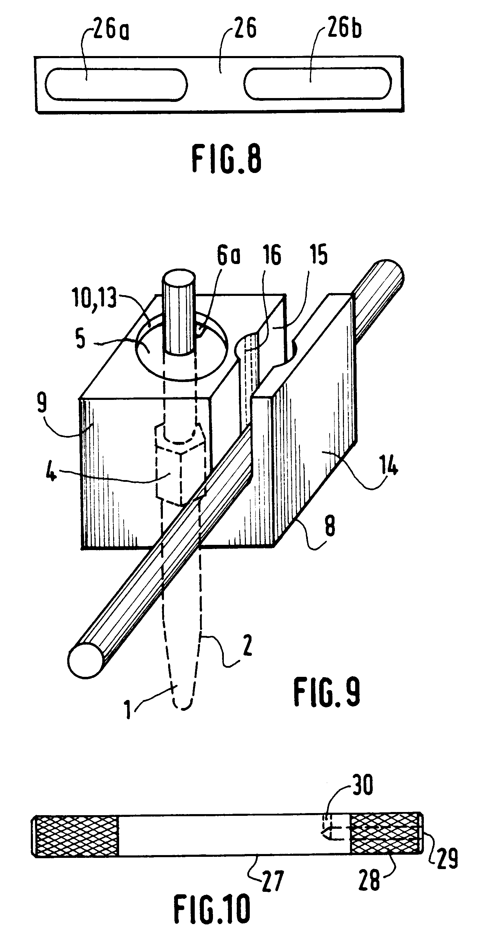

La figure 8 est une vue d'une plaque transversale selon la présente invention. Cette plaque 26 comporte à chacune de ses extrêmités deux trous, respectivement 26a et 26b, de forme globalement ovalaire. Cette plaque a une forme générale et est réalisée dans un matériau qui permet son adaptation à l'anatomie de la vertèbre.

La figure 9 est une vue en perspective du système d'ostéosynthèse selon la présente invention, tel que monté sur une vertèbre et au stade où la vis sans tête doit être mise en place pour effectuer la solidarisation du système. Les pointillés indiquent la partie de la vis pédiculaire cachée par la platine ou dans l'os vertébral.

Les dimensions de la vis sont classiques pour les systèmes d'ostéosynthèse rachidienne classiques. Ainsi, pour la vis, le filetage pédiculaire intra-osseux est d'une longueur d'environ 35 à environ 45 mm, le diamètre varie d'environ 3,5 à environ 5,5 mm pour la partie cylindrique la plus à l'intérieur de l'os et d'environ 4,5 à environ 6,5 mm pour la partie conique proche de la surface de l'os, lorsque la vis pédiculaire présente le profil tel que décrit à la figure 1. Le filetage de platine extra-osseux est d'une longueur d'environ 20 mm, parfois plus pour bénéficier d'un effet de tire-fond plus prononcé, et le diamètre varie entre environ 3,5 et environ 5,5 mm. Le moyen de serrage 4, l'élément à 6 pans plats représente environ 5 mm de la longueur du filetage de platine. En ce qui concerne la platine, les dimensions hors tout de celle-ci sont, à titre d'exemple: hauteur d'environ 10 mm, largeur d'environ 10 mm, longueur d'environ 15 mm. Lorsque l'alésage est en forme de U, le diamètre intérieur de celui-ci est d'environ 3 mm, soit environ le diamètre de la tige. Lorsque l'alésage a la forme d'un oméga, l'ouverture est sensiblement identique au diamètre de la tige. Le trou au travers de la tête s'engageant sur le filetage de platine est situé à environ 5 mm du bord de la platine, tandis que l'alésage est placé à environ 3,5 mm du bord de la platine et est pratiqué jusqu'à environ la moitié de l'épaisseur de la platine. Le filetage dans l'alésage a un diamètre d'environ 4 mm et est pratiqué de la surface de la platine jusqu'à atteindre l'alésage. La vis de blocage a ainsi une épaisseur d'environ 4 mm. La tige a un diamètre d'environ 3 mm et une longueur comprise entre 100 et 400 mm. L'épaisseur de l'écrou de serrage est de l'ordre de 4 mm, les rainures latérales ayant une profondeur d'environ 1 mm. Les dimensions de la plaque transversale sont standard; à titre d'exemple, la longueur est comprise entre environ 65 et environ 75 mm, la largeur est d'environ 8 mm, et la plus grande dimension des trous ovalaires est comprise entre environ 25 et environ 30 mm, la plus petite dimension est d'environ 4 mm et les trous sont situés à environ 3 mm du bord de la plaque transversale.

Le présent système d'ostéosynthèse rachidienne est mis en place à l'aide d'un matériel ancillaire qui comprend: pinces à distraction et compression, porte-tige, tubes de réduction, tournevis.

La figure 10 illustre un élément caractéristique de ce matériel, le tube de réduction 27. Celui-ci possède une extrémité distale 28 qui s'adapte sur l'extrémité libre du filetage de platine de la vis, éventuellement sur l'écrou. Cette extrêmité distale comporte un filetage 29 interne qui correspond au filetage de platine. Un trou de nettoyage 30 est par ailleurs prévu latéralement. Outre sa fonction propre, ce tube permet l'action des pinces réductrices dont le mors se compose d'une partie concave qui vient s'adapter au tube de réduction. Ces pinces de réduction sont de deux types, les unes permettant par leur serrage une compression, les autres permettant une distraction. Les pinces à compression peuvent aussi être adaptées sur les deux vis d'une même vertèbre et être utilisées en dérotation. Lorsque le système de blocage de la tige dans l'alésage est constitué par des clavettes, la pince porte-clavette possède une partie distale dont les extrémités des branches possèdent des crochets permettant d'exercer des contraintes sur la clavette. Tout matériel ancillaire classique peut par ailleurs être utilisé.

Le présent système d'ostéosynthèse peut être réalisé en tout matériau biocompatible classiquement utilisé dans l'art des prothèses, orthèses, ostéosynthèses et autres. A titre d'exemple de matériau présentant les qualités mécaniques et de biocompatibilité requises, on peut citer à titre non-limitatif les alliages de titane. De préférence, le matériau constitutif du présent système d'ostéosysthèse est un alliage à base de titane, avantageusement le TA6V.

La présente invention relative à un nouveau système d'ostéosynthèse rachidienne est décrite dans les exemples suivants, donnés à titre illustratif et nullemnt limitatifs.

### EXEMPLE 1: Spondylolisthésis

Les vis spéciales tire-fond, c'est-à-dire à filetage de platine d'une longueur plus grande, sont placées sur la vertèbre listhésique. Dans un premier temps, le chirurgien exerce une distraction sur les vertèbres sus- et sous-jacentes. Il est alors possible d'exercer une traction dans l'axe de la vertèbre intéressée, soit par serrage de l'écrou sur la platine après mise en place de la tige, soit à l'aide d'un tube réducteur muni d'un filetage correspondant à celui du filetage de platine de la vis pédiculaire, et solidarisation subséquente de l'ensemble.

### EXEMPLE 2: Scoliose lombaire courte dégénérative

Les vis pédiculaires sont posées sur les vertèbres stratégiques telles que L5, les platines correspondantes sont mises en place ainsi que les plaques transversales éventuelles, à chaque étage, l'ensemble est bloqué à l'aide d'un écrou.

Les tubes de réduction sont placés sur chaque vis et les pinces sont alors utilisées. Le chirurgien exerce une action proximale des pinces de distraction et il y a création de l'écart intervertébral. Ensuite, il exerce une action des pinces de compression en position transversale permettant la dérotation manuelle. Enfin, il exerce une action des pinces de compression sur la partie distale des tubes, assurant ainsi la mise en lordose. Les pinces sont alors "clampées", la réduction est maintenue ainsi, et les tiges de fixation peuvent être modelées et posées dans les gorges en U (respectivement en Ω (oméga)). Les vis sans tête (respectivement les clavettes) sont mises en place à chaque étage, et bloquées. Avant ce serrage définitif, dans la mesure où le diamètre intérieur de la gorge peut être supérieur à celui de la tige, il existe une possibilité d'appliquer un complément de dérotation, afin d'affiner le positionnement. Ceci est encore un avantage de la présente invention. La technique est simplifiée dans le cas des fractures, où la dérotation n'est généralement pas utile, et où la distraction et mise en lordose sont suffisantes.

## Revendications

1. Système d'ostéosynthèse rachidienne comprenant:
- une vis pédiculaire (1) munie de deux filetages, le premier filetage (2), le filetage pédiculaire, étant destiné à être introduit dans le pédicule vertébral et le second filetage (3), le filetage de platine, étant destiné à recevoir la platine (8);
- une tige (22);
- au moins une platine (8) avec au moins une tête (9) perforée d'un trou (10) dont le diamètre est sensiblement égal à celui du filetage de platine de la vis (1) pédiculaire ;
- un moyen de fixation (5) de la platine (8) sur la vis (1) destiné à solidariser ladite platine (8) au niveau de la tête (9) sur ladite vis pédiculaire (1);
- lesdits moyen de fixation (5) et filetage (3) de platine étant tels que le moyen de fixation (5) engagé à blocage laisse libre une partie du filetage (3) de platine;
caractérisé
en ce que ladite platine (8) est consituée d'au moins la tête et d'au moins une gorge (14) dont l'axe est sensiblement perpendiculaire à l'axe de la tête, ladite gorge (14) comprenant un alésage fendu (15) dont l'espace de l'ouverture correspond au moins au diamètre de la tige (22), la tige (22) étant susceptible d'être insérée dans l'alésage fendu dans une direction sensiblement perpendiculaire à l'axe longitudinal de la tige (22);
en ce que ledit moyen de fixation (5) et ledit filetage de platine (3) sont tels que le moyen de fixation engagé à blocage laisse libre une partie du filetage de platine permettant la fixation sur l'extrémité libre du filetage (3) d'un matériel ancillaire utilisé pour une action mécanique de réduction, et
en ce qu'il comprend un moyen (17) de fixation de la tige (22) dans la gorge (14) destiné à solidariser ladite tige (22) dans ladite gorge (14) de ladite platine (8).

2. Le système d'ostéosynthèse rachidienne selon la revendication 1, dans lequel l'alésage fendu (15) a la forme d'un U.

3. Le système d'ostéosynthèse rachidienne selon la revendication 1 ou 2, dans lequel le moyen de fixation (17) de la tige dans la gorge est une vis s'engageant dans un filetage (16) ménagé dans l'alésage (15) de la gorge (14,) sensiblement perpendiculairement à cette dernière et sensiblement parallèlement à la vis pédiculaire (1).

4. Le système d'ostéosynthèse rachidienne selon la revendication 3, dans lequel la vis (17) de fixation est une vis sans tête.

5. Le système d'ostéosynthèse rachidienne selon la revendication 1, dans lequel l'alésage fendu (15) a la forme d'un Ω (oméga).

6. Le système d'ostéosynthèse rachidienne selon la revendication 5, dans lequel le moyen (17) de fixation de la tige (22) dans la gorge (14) est une clavette (23) excentrique présentant une face interne dont le diamètre est substantiellement identique à celui de la tige et une face convexe (25) telle qu'une action mécanique de contrainte sur la platine (8) est exercée lors de la rotation de ladite clavette (23) autour de la tige (22) dans l'alésage fendu (15).

7. Le système d'ostéosynthèse rachidienne selon l'une quelconque des revendications 1 à 6, dans lequel le moyen de fixation (5) de la platine sur la vis est un écrou.

8. Le système d'ostéosynthèse rachidienne selon la revendication 7, dans lequel l'écrou (5) possède des rainures (6) de guidage de l'instrument de serrage.

9. Le système d'ostéosynthèse rachidienne selon l'une quelconque des revendications 1 à 8, dans lequel la vis pédiculaire est munie d'un moyen de serrage (4).

10. Le système d'ostéosynthèse rachidienne selon la revendication 9, dans lequel le moyen de serrage (4) est un écrou hexagonal situé entre le filetage de platine et le filetage pédiculaire.

11. Le système d'ostéosynthèse rachidienne selon l'une quelconque des revendications 1 à 10, dans lequel la vis pédiculaire (1) est de section décroissante dans sa partie pédiculaire.

12. Le système d'ostéosynthèse rachidienne selon l'une quelconque des revendications 1 à 11, dans lequel le filetage pédiculaire (2) possède un profil à fond plat.

13. Le système d'ostéosynthèse rachidienne selon l'une quelconque des revendications 1 à 12, dans lequel la vis comporte une couronne externe (19) entre les filetages afin de placer la vis pédiculaire en butée sur le rachis.

14. Le système d'ostéosynthèse rachidienne selon l'une quelconque des revendications 1 à 13, dans lequel la tête de la platine comporte un méplat (21).

15. Le système d'ostéosynthèse rachidienne selon les revendications 13 et 14, dans lequel la vis pédiculaire comporte sur la couronne externe (19) un méplat (20) situé entre les deux filetages, lesdits méplats (20, 21) étant correspondants et pouvant s'engager afin d'empêcher toute rotation de la platine sur la vis.

16. Le système d'ostéosynthèse rachidienne selon les revendications 10 et 14, dans lequel ledit méplat (20) et au moins un pan de l'écrou hexagonal sont correspondants et peuvent s'engager afin d'empêcher toute rotation de la platine sur la vis.

17. Le système d'ostéosynthèse rachidienne selon la revendication 13, dans lequel la couronne externe (19) située entre les deux filetages de la vis pédiculaire est une couronne dentée s'engageant dans une couronne dentée correspondante sur la platine, afin d'empêcher toute rotation de la platine sur la vis.

18. Le système d'ostéosynthèse rachidienne selon l'une quelconque des revendications 1 à 17, comprenant en outre une plaque transversale (26) s'étendant entre deux vis pédiculaires, fixées solidairement sur celles-ci avec les deux platines correspondantes et les deux écrous correspondants, avant action mécanique de réduction.

19. Le système d'ostéosynthèse rachidienne selon l'une quelconque des revendications 1 à 18, dans lequel la platine possède deux gorges.

20. Le système d'ostéosynthèse rachidienne selon l'une quelconque des revendications 1 à 19, comprenant deux platines, inférieure et supérieure.

21. Le système d'ostéosynthèse rachidienne selon la revendication 20, dans lequel la platine inférieure possède au moins un méplat supérieur sur la tête, la platine supérieure possède un méplat inférieur, lesdits méplats supérieur de la platine inférieure et inférieur de la platine supérieure venant en engagement.

22. Le système d'ostéosynthèse rachidienne selon la revendication 20, dans lequel la platine inférieure possède au moins une couronne dentée supérieure sur la tête, la platine supérieure possède une couronne dentée inférieure, lesdites couronnes dentées supérieure de la platine inférieure et inférieure de la platine supérieure venant en engagement.

## Claims

1. A system for spinal osteosynthesis comprising:
- a pedicle screw (1) having two threaded portions, the first (2) of which, constituting a pedicle thread, is adapted to be introduced into the pedicle of a vertebra and the second threaded portion (3), constituting a saddle thread, is adapted to receive a saddle member;
- a rod (22);
- at least one saddle member (8) consisting of at least one head (9) provided with a hole (10) of a diameter substantially corresponding to the diameter of the saddle thread provided on said pedicle screw (1);
- means (5) for securing said saddle member (8) on said screw (1) and adapted to render said saddle member (8) at the head (9) thereof integral with said pedicle screw (1);
- said securing means (5) and said saddle thread (3) being such that when said securing means (5) is engaged in its locked position, a part of said saddle thread (3) is left free;
characterized:
- in that said saddle member (8) is constituted by at least the head and at least one throat portion (14) the axis of said throat portion being substantially perpendicular to the axis of said head, said throat portion (14) including a split passage (15) the size of the opening of which corresponds at least to the diameter of said rod (22), said rod (22) beind adapted to be introduced into said split passage in a direction substantially perpendicular to the longitudinal axis of said rod (22);
- in that said securing means (5) and said saddle thread (3) are such that when said securing means is engaged in its locked position, a part of said saddle thread is left free allowing ancilliary equipment used for the mechanical action of reduction to be fixed onto the free end of said thread (3); and
- in that it comprises means 17) for securing said rod (22) in said throat portion (14) and for rendering said rod (22) integral with said throat portion (14) of said saddle member (8).

2. The system for spinal osteosynthesis according to claim 1, wherein said split passage (15) is generally U-shaped in cross-section.

3. The system for spinal osteosynthesis according to claim 1 or 2, wherein said means (17) for securing said rod in said throat portion comprises a screw engaging a threaded portion (16) provided in the passage (15) of said throat portion (14) substantially perpendicular thereto and substantially parallel to said pedicle screw.

4. The system for spinal osteosynthesis according to claim 3, wherein said screw (17) for securing said rod in said throat portion is a grub screw.

5. The system for spinal osteosynthesis according to claim 1, wherein the cross section of said split passage (15) is generally Ω (omega) -shaped.

6. The system for spinal osteosynthesis according to claim 5, wherein said means (17) for securing said rod (22) in said throat portion (14) is an excentric key (23) having an inner face the diameter of which is substantially equal to the diameter of the rod and a convex face (25) whereby mechanical stress is applied to said saddle member (8) upon rotation of said key (23) around the rod (22) in said split passage (15).

7. The system for spinal osteosynthesis according to any one of claims 1 to 6, wherein said means (5) for securing said saddle member on said screw is a nut.

8. The system for spinal osteosynthesis according to claim 7, wherein said nut (5) includes grooves (6) for guiding a tool adapted to tighten it.

9. The system for spinal osteosynthesis according to any one of claims 1 to 8, wherein the pedicle screw further comprises tightening means (4) between the said pedicle thread and the said saddle thread for screwing the pedicle screw into the vertebra.

10. The system for spinal osteosynthesis according to claim 9 wherein said tightening means (4) is a hexagonal nut located between the saddle thread and pedicle head.

11. The system for spinal osteosynthesis according to any one of claims 1 to 10, wherein said pedicle screw (1) has a diminishing cross-section in the pedicle thread portion thereof.

12. The system for spinal osteosynthesis according to any one of claims 1 to 11, wherein the bottom portion of the thread profile of said pedicle thread (2) is flat.

13. The system for spinal osteosynthesis according to any one of claims 1 to 12, wherein said pedicle screw includes an external crown (19) between the said two threaded portions thereof whereby the screw comes into abutment with the spine.

14. The system for spinal osteosynthesis according to any one of claims 1 to 13, wherein the head of said saddle member includes at least one flat (21).

15. The system for spinal osteosynthesis according to claims 13 and 14, wherein the outer crown (19) of said pedicle screw includes a flat (20) between the two said threaded portions, the said two flats (20, 21) having corresponding shapes and engaging with each other in order to prevent any possibility of said saddle member rotating on said screw.

16. The system for spinal osteosynthesis according to claims 13 and 14, wherein said flat (20) and at least one flat of the hexagonal screw have corresponding shapes and can engage with each other in order to prevent any possibility of said saddle member rotating on said screw.

17. The system for spinal osteosynthesis according to claim 10 and 14, wherein said outer crown (19) situated between the said two threaded portions of the pedicle screw is a toothed crown which engages with a corresponding toothed crown on said saddle member in order to prevent any possibility of said saddle member rotating on said screw.

18. The system for spinal osteosynthesis according to any one of claims 1 to 17, further comprising a transverse plate (26) extending between two pedicle screws and rigidly fixed thereon together with the two corresponding saddle members and nuts, prior to the application of mechanical force for reduction purposes.

19. The system for spinal osteosynthesis according to any one of claims 1 to 18 in which said saddle member includes two throat portions.

20. The system for spinal osteosynthesis according to any one of claims 1 to 19, wherein said system includes two saddle members consisting of an upper saddle member and a lower saddle member.

21. The system for spinal osteosynthesis according to claim 20, in which said lower saddle member includes at least one flat on its head and the upper saddle member includes a lower flat, said upper flat on the lower saddle member and lower flat on the upper saddle member engaging with each other.

22. The system for spinal osteosynthesis according to claim 20, in which said lower saddle member includes at least one toothed crown on its head and the upper saddle member includes a lower toothed crown, said upper toothed crown on the lower saddle member and lower toothed crown on the upper saddle member engaging with each other.

## Patentansprüche

1. Wirbelsäulenosteosynthesesystem, umfassend:
- eine Bogenfußschraube (1), die mit zwei Gewinden versehen ist, wobei das erste Gewinde (2), das Bogenfußgewinde, vorgesehen ist, um in den Wirbelbogenfuß eingeführt zu werden, und wobei das zweite Gewinde (3), das Platinengewinde, vorgesehen ist, die Platine (8) aufzunehmen;
- einen Schaft (22);
- zumindest eine Platine (8) mit zumindest einem durch ein Loch (10) perforierten Kopf, dessen Durchmesser im wesentlichen demjenigen des Platinengewindes der Bogenfußschraube (1) entspricht;
- eine Befestigungseinrichtung (5) für die Platine (8) an der Schraube (1), vorgesehen, um mit der Platine (8) auf der Höhe des Kopfes (9) an der Bogenfußschraube (1) befestigt zu werden; wobei die Befestigungseinrichtung (5) und das Platinengewinde (3) in solch einer Weise ausgebildet sind, daß die Befestigungseinrichtung (5) unter Blockageeingriff einen Abschnitt des Platinengewindes (3) freiläßt;
dadurch gekennzeichnet, daß
die Platine (8) gebildet ist aus zumindest dem Kopf und zumindest einer Aussparung (14), deren Achse im wesentlichen senkrecht zu der Achse des Kopfes verläuft, wobei die Aussparung (14) eine geschlitzte Bohrung (15) umfaßt, deren Öffnungsraum zumindest dem Durchmesser des Schaftes (22) entspricht, wobei der Schaft (22) in der Lage ist, in die geschlitzte Bohrung in einer Richtung im wesentlichen senkrecht zu der Längsachse des Schaftes (22) eingeführt zu werden;
und daß die Befestigungseinrichtung (5) und das Platinengewinde (3) in solch einer Weise ausgebildet sind, daß die Befestigungseinrichtung in Blockageeingriff einen Teil des Platinengewindes freiläßt, so daß an dem freien Ende des Gewindes (3) ein Zusatz- bzw. Hilfsmaterial befestigt werden kann, welches für eine mechanische Reduktionswirkung verwendet wird, und daß sie ein Befestigungsmittel (17) des Schaftes (22) in der Aussparung (14) umfaßt, vorgesehen, um den Schaft (22) mit der Aussparung (14) der Platine (8) zu verbinden.

2. Wirbelsäulenosteosynthesesystem nach Anspruch 1, in welchem die geschlitzte Bohrung (15) eine U-Form aufweist.

3. Wirbelsäulenosteosynthesesystem nach Anspruch 1 oder 2, in welchem das Befestigungsmittel (17) des Schaftes in der Aussparung eine Schraube ist, die mit einem Gewinde (16) in Eingriff bringbar ist, welches in der Bohrung (15) der Aussparung (14) ausgebildet ist, und zwar im wesentlichen senkrecht bezüglich der letzteren und im wesentlichen parallel zu der Bogenfußschraube (1).

4. Wirbelsäulenosteosynthesesystem nach Anspruch 3, in welchem die Befestigungsschraube (17) eine Madenschraube ist.

5. Wirbelsäulenosteosynthesesystem nach Anspruch 1, in welchem die geschlitzte Bohrung (15) eine Ω-Form (Omega) aufweist.

6. Wirbelsäulenosteosynthesesystem nach Anspruch 5, in welchem das Befestigungsmittel (17) des Schaftes (22) in der Aussparung (14) eine exzentrische Nocke (23) ist, die eine Innenfläche aufweist, wobei der Durchmesser im wesentlichen identisch zu jenem des Schaftes ist, sowie eine konvexe Fläche (25), so daß eine mechanische Spannwirkung an der Platine (8) ausgeübt wird, wenn die Nocke (23) um den Schaft (22) in der geschlitzten Bohrung (15) gedreht wird.

7. Wirbelsäulenosteosynthesesystem nach einem der Ansprüche 1 bis 6, bei welchem die Befestigungseinrichtung (5) der Platine an der Schraube eine Mutter ist.

8. Wirbelsäulenosteosynthesesystem nach Anspruch 7, in welchem die Mutter (5) Führungsrillen (6) für das Einspanninstrument umfaßt.

9. Wirbelsäulenosteosynthesesystem nach einem der Ansprüche 1 bis 8, in welchem die Bogenfußschraube mit einer Spann- bzw. Einspann- bzw. Festzieheinrichtung (4) versehen ist.

10. Wirbelsäulenosteosynthesesystem nach Anspruch 9, in welchem die Spanneinrichtung (4) eine hexagonale Mutter ist, die zwischen dem Platinengewinde und dem Bogenfußgewinde angeordnet ist.

11. Wirbelsäulenosteosynthesesystem nach einem der Ansprüche 1 bis 10, in welchem die Bogenfußschraube (1) in dem Bogenfußabschnitt einen sich verjüngenden bzw. abnehmenden Schnitt aufweist.

12. Wirbelsäulenosteosynthesesystem nach einem der Ansprüche 1 bis 11, in welchem das Bogenfußgewinde (2) ein Profil mit ebenem Grund bzw. Boden aufweist.

13. Wirbelsäulenosteosynthesesystem nach einem der Ansprüche 1 bis 12, in welchem die Schraube eine externe Krone (19) zwischen den Gewinden umfaßt, um die Bogenfußschraube anliegend an der Wirbelsäule anzuordnen.

14. Wirbelsäulenosteosynthesesystem nach einem der Ansprüche 1 bis 13, in welchem der Kopf der Platine eine Abflachung (21) umfaßt.

15. Wirbelsäulenosteosynthesesystem nach den Ansprüchen 13 und 14, in welchem die Bogenfußschraube an der externen Krone (19) eine Abflachung (20) umfaßt, angeordnet zwischen den zwei Gewinden, wobei die Abflachungen (20, 21) einander entsprechen und in Eingriff bringbar sind, um jegliche Rotation der Platine an der Schraube zu verhindern.

16. Wirbelsäulenosteosynthesesystem nach den Ansprüchen 10 und 14, in welchem die Abflachung 20 und zumindest eine Kante bzw. Fläche der sechseckigen bzw. hexagonalen Mutter einander entsprechend ausgebildet sind und in Eingriff treten können, um jegliche Rotation der Platine an der Schraube zu verhindern.

17. Wirbelsäulenosteosynthesesystem nach Anspruch 13, in welchem die externe Krone (19), die zwischen den zwei Gewinden der Bogenfußschraube angeordnet ist, eine gezahnte Krone ist, in Eingriff bringbar mit einer entsprechenden gezahnten Krone an der Platine, um jegliche Rotation der Platine an der Schraube zu verhindern.

18. Wirbelsäulenosteosynthesesystem nach einem der Ansprüche 1 bis 17, ferner umfassend eine Transversalplakette (26), die sich zwischen zwei Bogenfußschrauben erstreckt, wobei fest an diesen mit den zwei entsprechenden Platinen die zwei entsprechenden Muttern befestigt sind, und zwar vor einer mechanischen Reduktionswirkung.

19. Wirbelsäulenosteosynthesesystem nach einem der Ansprüche 1 bis 18, in welchem die Platine zwei Aussparungen aufweist.

20. Wirbelsäulenosteosynthesesystem nach einem der Ansprüche 1 bis 19, umfassend zwei Platinen, nämlich eine untere und eine obere.

21. Wirbelsäulenosteosynthesesystem nach Anspruch 20, in welchem die untere Platine zumindest eine obere Abflachung an dem Kopf aufweist, wobei die obere Platine eine untere Abflachung aufweist, wobei die obere Abflachung der unteren Platine und die untere Abflachung der oberen Platine in Eingriff bringbar sind.

22. Wirbelsäulenosteosynthesesystem nach Anspruch 20, in welchem die untere Platine zumindest eine gezahnte obere Krone an dem Kopf umfaßt, wobei die obere Platine eine gezahnte untere Krone umfaßt, wobei die gezahnte obere Krone der unteren Platine und die gezahnte untere Krone der oberen Platine in Eingriff bringbar sind.
